# EUROPEAN PATENT APPLICATION

(11) **EP 2 238 935 A2**
(43) Date of publication of application: **13.10.2010**
(21) Application number: 10170527.5
(22) Date of filing: 10.02.2004
(51) Int. Cl.: A61B 17/70, F16B 33/02, F16B 35/04

(54) **Implant device including threaded locking mechanism**

(30) Priority: 19.02.2003 US 369158
(62) Divisional of application: 04250700.4
(71) Applicant: Aesculap Implant Systems, Inc., Center Valley, PA 18034 (US)
(72) Inventor: Raynor, Donald E, Burlison, TN 38015 (US); Adams, James W, Atoka, TN 38004 (US)
(74) Representative: Wallis, Naomi Rachel

(57) **Abstract**

A medical device assembly includes a rod receiving member for receiving a portion of a rod member therein. A locking member locks the rod member in place in the rod receiving member. A gripping mechanism between threads in the rod receiving member and threads in the locking member prevent splaying of the threads when the threads are threadedly entrained together.

## Description

### BACKGROUND OF THE INVENTION

### 1. TECHNICAL FIELD

The present invention relates to screw thread designs. More specifically, the present invention relates to screws disposed in thin walled retainers, and especially for use in medical devices.

### 2. BACKGROUND ART

In various industries, threaded devices, such as set screws, are commonly used. Set screws are used in many environments in order to lock one element of a device relative to another. Set screws are quite important in the art of medical implants, as it is often necessary to capture one element of the implant relative to another and to then lock the two relative to one another to prevent subsequent movement therebetween. Failure to properly lock two elements of a medical implant together may result in failure of the implant and possible serious injury to the patient within which the implant is placed. For example, in orthopedic devices, a rod is often loaded into a recess or channel of a retaining member, such as a fixation element or the like. Such a device is disclosed in United States Patent No. 6,296,642 to Morrison, et al., issued October 2, 2001. As discussed in the Morrison, et al. patent, a difficulty that has been experienced in the orthopedic device industry, as well as in other non-related industries, is that the configuration of many devices requires a threaded device, such as a set screw, to be disposed between upright arms or thin walled sections of a body portion. These sections can experience splaying after implantation.

An inherent problem in certain devices, such as medical implants, with set screws of a conventional type is that such set screws typically utilize threads which are referred to as V-threads. The edges of a cross-section of V-threads have a V shape. V-threads work reasonably well in devices including a bore that completely surrounds the set screw and has a mating thread that mates with the thread of the set screw. As stated above, many devices, such as bone screws, do not provide for a bore that will entirely encircle the set screw. In such implants, the set screw also functions as a closure and spans between a pair of discontinuous threaded surfaces. When V-thread set screws are utilized for this purpose, the forces exerted by the set screw during torquing are partially parallel to the axis of rotation of the set screw and partially radially extending outwardly from the set screw. These radial outward forces can and frequently do spread the discontinuous threaded surfaces which causes failure of the threaded locking mechanism. The resulting splaying of the discontinuous threaded surface is a major problem.

To prevent splaying, prior medical devices have included a nut, cap, clamp or similar apparatus to surround and hold the legs of the fixation element together. For example, in U.S. Patent No. 5,672,176 to Biedermann, et al., a rod is placed into a slot in the fixation element. The locking member is engaged with the fixation element to press down via an intermediary part on the rod. An outer nut is threaded on the outside of the fixation element. Although effective in controlling splaying, these devices have tended to be relatively more expensive and less efficient to implant compared with devices without an outer nut or cap. The outer nut or cap also adds to the profile of the medical device, making the device more difficult to implant in the frequently limited area in which to perform surgery and/or place an implant. A larger implant can also result in a higher risk of residual pain to the patient or potential complications.

Buttress-type threads have been utilized for the purpose of trying to reduce the radial outward forces that are exerted by the threads. In buttress-type thread screws, the trailing surface of the thread normally has a cross-section edge that is parallel to or is fairly close to being parallel to a radius of the set screw. Sometimes such surfaces are referred to as flat, but normally the cross section has a slight inclination of from 5 to 10 degrees so that a smaller, but yet substantial force, is exerted radially outward by the buttress thread screws as compared to the V-shaped thread screws. Consequently, it is desirable to also have a set screw of this type wherein the threads are designed to exert an inwardly directed force to pull opposing walls of an implant toward the set screw, rather than urge the walls away from the set screw.

It is also necessary for the set screw to tightly grip whatever element it is urged against so as to lock that element relative to a second element within which the set screw is threaded. Such locking is partially provided by friction. Positive penetration of the set screw into the element to be set assists in the locking and provides for a more secure lock. A smooth circular surface on the underside of the set screw does not provide digging into or abrasion of the element to be locked and such smooth bottom set screws must rely solely upon the friction generated between a fairly smooth surface and the other element, such as a rod for secure locking. In order to overcome this problem, the prior art has utilized various structures on the end of the set screw such as points, knurling and cutting rings.

There is therefore a need remaining in industry, especially for medical devices, and particularly orthopedic devices, which minimize the profile and bulk of the components of the device and minimizes the cost and difficulty of using such devices, while still preventing splaying of the fixation elements.

### SUMMARY OF THE INVENTION

According to the present invention, there is provided a medical device including a rod receiving mechanism for receiving a portion of a rod member therein and a locking mechanism for locking the rod member in place in the rod receiving mechanism. A gripping mechanism grips a first thread of the rod receiving mechanism with a second thread of the locking mechanism when the threads are threadedly engaged and prevents splaying of the threads when the threads are threadedly entrained together.

### DESCRIPTION OF THE DRAWINGS

Other advantages of the present invention are readily appreciated as the same becomes better understood by reference to the following detailed description when considered in connection with the accompanying drawings wherein:
Figure 1 is a prospective view of the medical device of the present invention;
Figure 2 is a side view of the receiving member of the present invention;
Figure 3 is an enlarged view of the thread of the receiving member shown in Figure 2;
Figures 4 A-F are cross-sectional views of variations of the thread made in accordance with the present invention;
Figure 5 is an enlarged side view, partially broken away, of a screw and nut of the present invention; and
Figure 6 is a prospective schematic view of the screw of the present invention.

### DETAILED DESCRIPTION OF THE INVENTION

The present invention generally relates to a thread configuration for use in many applications, and with particular value in medical devices. The preferred embodiments discussed herein are surgical implants. However, the inventive thread configuration of the present invention possesses wide spread applicability beyond medical devices.

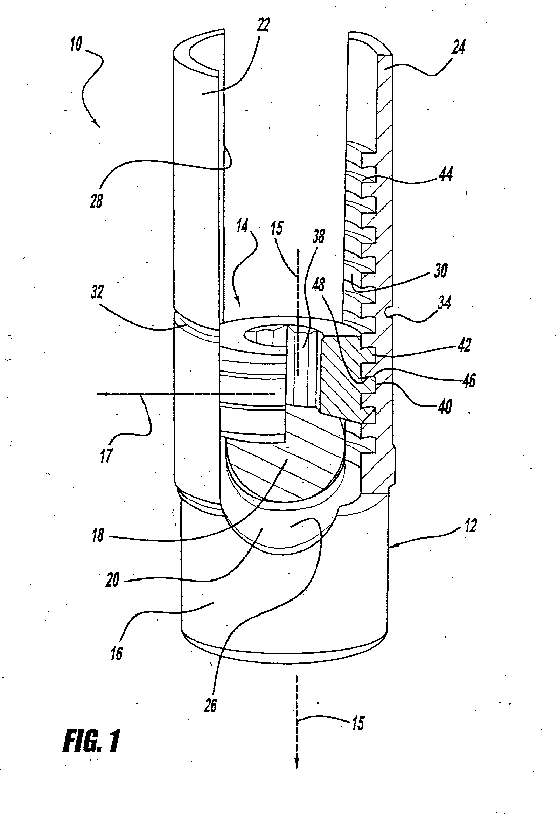

A preferred embodiment of the present invention in the environment of a medical device is generally shown at 10 in the Figures. The device 10 includes a rod receiving member generally indicated at 12 and a locking member in the form of a set screw generally indicated at 14. The threads of the rod receiving member 12 and set screw 14 grip each other and thereby prevent splaying of the threads when the threads are threadedly entrained together, as shown in Figure 1. This "gripping" between the threads of the rod receiving member 12 and set screw 14 eliminate or at least significantly reduce spreading of the thin walls of the rod receiving member 12, as explained in more detail below.

The term "gripping" means that the threads not only engage each other in a direction parallel to the longitudinal axis 15 of the set screw 14, but also engage each other in a direction 17 radially extending from the longitudinal axis 17. Thus, the set screw 14 is longitudinally fixed and the gripping of the threads prevents separation of the threads in a radial direction 17, relative to the longitudinal axis.

More specifically, a rod receiving member includes a body portion 16 including a substantially U-shaped recess for receiving a rod member 18 therein, as shown in Figure 1. The substantially U-shaped recess is defined by a base portion 20 of the recess and a pair of arms 22, 24. The base portion 20 includes an arcuate surface 26, which is substantially smooth. Each of the arms 22, 24 includes a threaded inner surface 28, 30, respectively.

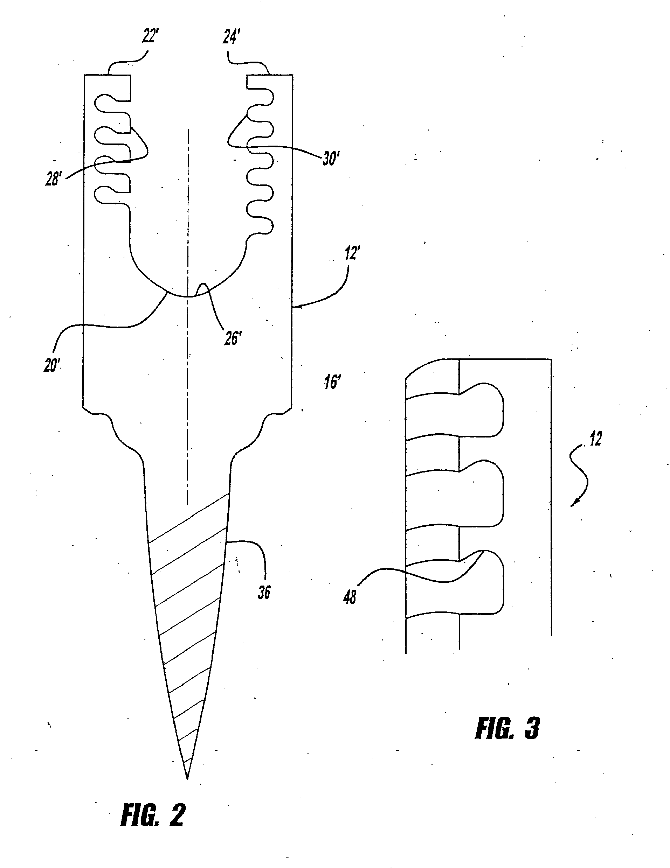

The rod receiving member can assume various specific forms, two of which being shown in Figures 1 and 2. In Figure 1, the locking member includes extended arms 22 and 24, each of the arms 22, 24 including an indent or recess 32, 34 allowing for easy breaking off of the extended portion of the arms 22, 24. The extended arms 22, 24 allow for easier access of the set screw 14. Once the set screw 14 is in place, that being in locked condition against the rod 18 disposed within the recess of the rod retaining member 12, then the indents 32, 34 allow for easy breaking off of the extended portion of the arms 22, 24. In this manner, the profile of the final assembly 10 is much smaller and more adaptable to remain in place within the patient.

In Figure 2, like structures between the two embodiments are shown by prime numbers. The rod receiving member 12' includes an integral threaded screw portion 36 for direct engagement with a bone, such as a vertebrae. Other multiple piece embodiments, such as those shown in United States Patent No. 5,964,760 to Richelsoph, and assigned to the assignee of the presently pending application, can also be used, such as set screws having spherical heads and the rod retaining member 12 including a receiving portion for variable angular adjustment relative to the screw head. In other words, the present invention can be used with various modifications of the rod receiving member that are well known in the art.

The set screw 14 includes a body portion 38 having a threaded outer surface 40 as shown in Figure 1. The threaded outer surface 40 of the screw member is in threaded engagement with the threaded inner surfaces 28, 30 of the arms 22, 24 so as to be screwed into locked engagement with the rod member 18 seated against the arcuate surface 26 of the base portion 20. The threaded surface 40 includes threads 42, which grip in mating engagement with the threads 44 of the arms 22, 24. This "gripping" prevents the arms 22, 24 from splaying from or being ripped away from engagement with the threads of the set screw 14.

More specifically, the threads 42, 44 of the present invention generally include a curved portion which mates and engages a curved portion of a mating thread. Such curvatures can be inverted and function in accordance with the present invention. However, there are benefits, discussed below, derived from the rod receiving member 12 including internal threads 44 and a curved, concave portion cut into a recess which forms the remainder of the thread. The curved portion is upwardly facing away from the seat of the screw member 38. This curvature provides that the curved portion of the internal thread will engage the external curved portion of the set screw member 38 under load conditions.

Figure 1 shows the assembled device 10 retaining the rod member 18. The set screw 38 is engaged in the rod receiving member 12 of the implant assembly 10. The concave portion 46 of the thread 40 of the set screw 38 engages the convex portion 48 of the thread 40 of the arms 22, 24. Since the convex portion 48 extends well into the concavity, the engagement can eliminate, or at least significantly reduce spreading of the thin walled arms 22, 24 (radially outward extension of arms 22, 24), thereby reducing or solving a significant problem of the prior art.

Also of importance is the friction reduction value of the thread of the present invention. Since the thread engagement surface is a rounded bearing surface, the friction is reduced over threads existing in the prior art. As friction is a major factor in reducing screw thread-type connector efficiency, reducing friction increases the amount of available energy to lock the assembly. Since the amount of force it takes to lock an implant with existing technology and threads can be very high, it is highly advantageous to find a more efficient thread form. The unique thread form of the present invention solves many of the issues and problems associated with existing thread technology.

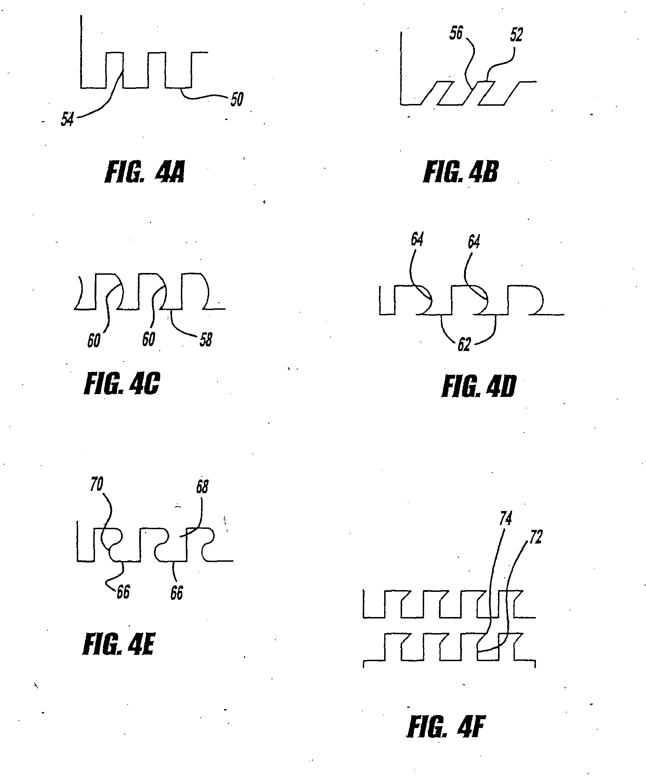

Figures 4 A-F shows various permutations of the present invention. Prior art threads as shown in Figures A and B include teeth 50, 52 having straight-sided walls 54, 56 respectively. There is no gripping of such teeth when entrained or threaded into mating engagement with a receiving thread.

Figures C, D, E, and F in Figure 4 are examples of various teeth configurations which allow for the gripping function of the present invention. Figure 4 C shows teeth 58 having radius sides 60. Such teeth do not require great depth in order to function in accordance with the present invention. Figure 4 D shows teeth 62 having a side 64. Each side 64 has a radius with a height of cord in its center. In this manner, forces are kept outside the vertical and the teeth ride on their bearing surfaces. Such teeth configuration does not require great depth and leaves maximum wall strength for the body, the teeth locking on their radius. Figure 4 E shows teeth 66 having what is referred to as a dog bone configuration. The teeth 66 include a thinner base portion 68 and a projecting portion at the end of the tube 70 for mating engagement in a like recess a mating thread. The dog bone configuration can have an end portion with a radius as shown in Figure 4 E or such a thread 72 can have an angled end portion 74 as shown in Figure 4 F.

In operation, the rod retaining member is affixed *in situ* by means well known in the art. For example, a rod retaining member 12' shown in Figure 2 can be threadedly engaged or screwed into a vertebra by a practitioner. The portion of the rod member 18 is disposed within the recess of the rod retaining member 12 as shown in Figure 1 and the set screw 38 screwed into engagement and locked against the rod member 18. The mating curved thread 40 of the set screw member 38 grips the curvature of the inner aspect of the thread 28, 30 of the arms 22, 24. In this manner, the set screw member 38 functions to 1) lock the rod member 18 in place; 2) grip the threads of the retaining member 12, prevent splaying of the arms 22, 24 relative to the set screw member 38; and 3) provide increased efficiency during the screwing and locking process.

The present invention has widespread use in many industries. The goal of many industries is to reduce weight of devices and/or cost of production. For example, the automotive industry over the past twenty years has been constantly moving towards thin walled components. The above discussed issue of splaying is a great concern where various threaded elements are disposed within retainers wherein the retainer is a thin walled component. The present invention has great applicability in such uses, thereby eliminating weight of components, the need of extra components to prevent splaying, etc.

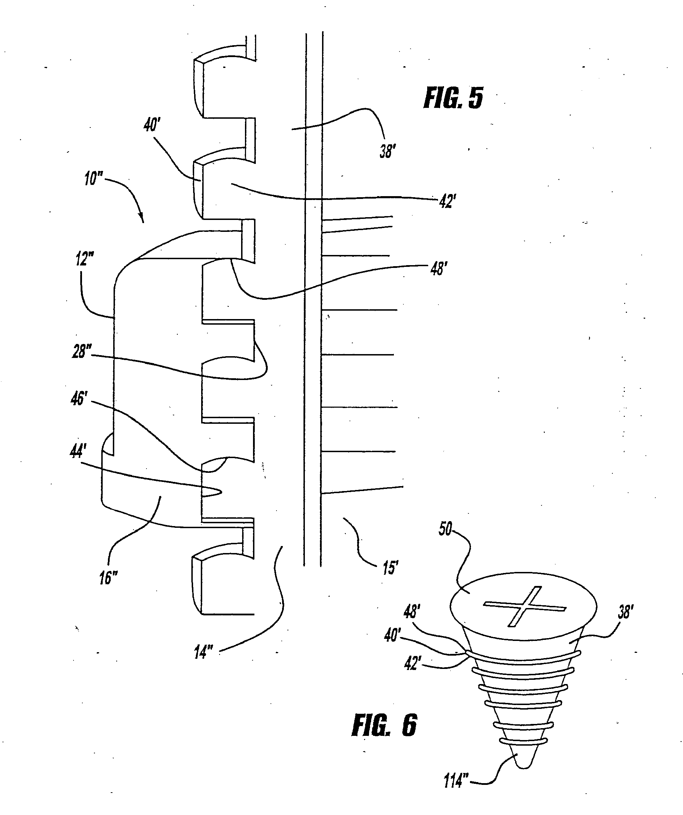

In an alternative embodiment of the present invention, the device 10 includes a nut 12" and a locking member in the form of a screw generally indicated at 14". The threads of the nut 12" and screw 14" grip each other and thereby prevent splaying of the threads when the threads are threadedly entrained together, as shown in Figure 5.

The nut 12" includes a body portion 16" including a recess for receiving a screw 14" as shown in Figure 5. The nut 12" is shaped sufficiently to encompass the screw 14". The nut 12" includes a threaded inner surface 28". The threaded inner surface 28" includes threads 42' having a curved portion that mates and engages a curved portion of the mating thread. Such curvatures can be inverted and function in accordance with the present invention. In the preferred embodiment, the recess 42' includes a concave portion 46'.

The set screw 14" includes a body portion 38' having a threaded outer surface 40', as shown in Figure 6. The threaded outer surface 40' of the screw is in threaded engagement with the threaded inner surfaces 28" of the nut 12". The threaded surface 40' includes threads 42', which grip in mating engagement with the threads 44' of the nut 12". This "gripping" prevents the nut from splaying or being ripped away from engagement with the threads of the set screw 14".

Figure 5 shows the assembled device 10'. The set screw 14" is engaged in the nut 12". The concave portion 46' of the thread 40' of the set screw 38' engages the convex portion 48' of the thread 40' of the nut 12". Since the convex portion 48' extends well into the concavity, the engagement can eliminate, or at least significantly reduce spreading of the nut 12".

Throughout this application, various publications, including United States patents, are referenced by author and year and patents by number. Full citations for the publications are listed below. The disclosures of these publications and patents in their entireties are hereby incorporated by reference into this application in order to more fully describe the state of the art to which this invention pertains.

The invention has been described in an illustrative manner, and it is to be understood that the terminology, which has been used is intended to be in the nature of words of description rather than of limitation.

Obviously, many modifications and variations of the present invention are possible in light of the above teachings. It is, therefore, to be understood that within the scope of the appended claims, the invention can be practiced otherwise than as specifically described.

### REFERENCES

### Foreign Patents:

DE 101 57 969 C1

### Clauses

1. A set screw comprising:
   a body portion having an outer surface and a first thread disposed on said outer surface, said thread including gripping means for gripping a second thread into which said first thread is threadedly entrained to prevent splaying of said two engaged threads.
2. The set screw according to clause 1 wherein said gripping means includes a curvate portion of said first thread.
3. The set screw according to clause 2 wherein said curvate portion extends over said entire first thread.
4. The set screw according to clause 2 wherein said curvate threads are in a shape selected from the group including a single curved side, two curved sides, and a dog bone configuration.
5. A receiver member comprising a body portion including a substantiated U-shaped recess for receiving a rod member therein, said recess including an internal threaded portion including gripping means for gripping a threaded portion of a set screw in threaded engagement engaged therewith to prevent splaying of said engaged threads.
6. The receiver member according to clause 5 wherein said gripping means includes a curvate portion of said first thread.
7. The receiver member according to clause 6, wherein said curvate portion extends over said entire first thread.
8. The receiver member according to clause 6, wherein said curvate threads are in a shape selected from the group including a single curved side, two curved sides, and a dog bone configuration.
9. A medical device comprising:
   a rod receiving means for receiving a portion of rod member therein and locking means for locking the rod member in place in said rod receiving means and gripping means for gripping a first thread of said rod receiving means with a second thread of said locking means when the threads are threadedly engaged and preventing splaying of said threads when said threads are threadedly entrained together.
10. The medical device according to clause 9 wherein said rod receiving means includes a body portion including a substantially U-shaped recess for receiving a rod member therein, said recess including an internally threaded portion including gripping means for gripping a threaded portion of a set screw in threaded engagement engaged therewith to prevent splaying of said engaged threads.
11. The medical device according to clause 9 wherein said device includes a set screw including said locking means, said set screw including a body portion having an outer surface, and a first thread disposed on said outer surface, said thread including gripping means for gripping a second thread into which said first thread is threadedly entrained to prevent splaying of said two engaged threads.
12. The medical device according to clause 9 wherein said rod receiving means includes a base portion and two arms extending therefrom defining a substantially U-shaped recess for receiving the portion of rod member therein, each of said arms including a threaded inner surface including said gripping means, said locking means being threadedly received between mid arms, said gripping means preventing spreading apart of said arms from said locking means when said locking means is threadedly engaged within said arms.
13. A curvate screw thread.
14. The curvate screw thread according to clause 13, wherein said curvate thread is an external thread.
15. The curvate screw thread according to clause 14, wherein said curvate thread is a convex external thread.
16. The curvate screw thread according to clause 13, wherein said curvate thread is on a male part.
17. The curvate screw thread according to clause 13, wherein said curvate thread is on a female part.
18. An anti-splay device comprising:
   a threaded body portion, at least a portion of said threaded body portion including a curvate thread.
19. A method of preventing splaying of two threadedly engaged components by gripping the engaged threads to each other.
20. An insert element comprising:
   a body portion having an outer surface and a first thread disposed on said outer surface, said thread including gripping means for gripping a second thread into which said first thread is threadedly entrained to prevent splaying of said two engaged threads.
21. An external nut having a curvate screw thread.

## Claims

1. A set screw comprising:
a body portion having an outer surface and a first thread disposed on said outer surface, said thread including gripping means for gripping a second thread into which said first thread is threadedly entrained to prevent splaying of said two engaged threads.

2. The set screw according to claim 1 wherein said gripping means includes a curvate portion of said first thread.

3. The set screw according to claim 2 wherein said curvate portion extends over said entire first thread.

4. The set screw according to claim 2 wherein said curvate threads are in a shape selected from the group including a single curved side, two curved sides, and a dog bone configuration.

5. A receiver member comprising a body portion including a substantiated U-shaped recess for receiving a rod member therein, said recess including an internal threaded portion including gripping means for gripping a threaded portion of a set screw in threaded engagement engaged therewith to prevent splaying of said engaged threads.

6. The receiver member according to claim 5 wherein said gripping means includes a curvate portion of said first thread.

7. The receiver member according to claim 6, wherein said curvate portion extends over said entire first thread.

8. The receiver member according to claim 6, wherein said curvate threads are in a shape selected from the group including a single curved side, two curved sides, and a dog bone configuration.

9. A curvate screw thread.

10. The curvate screw thread according to claim 9, wherein said curvate thread is an external thread.

11. The curvate screw thread according to claim 10, wherein said curvate thread is a convex external thread.

12. The curvate screw thread according to claim 9, wherein said curvate thread is on a male part.

13. The curvate screw thread according to claim 9, wherein said curvate thread is on a female part.

14. An anti-splay device comprising:
a threaded body portion, at least a portion of said threaded body portion including a curvate thread.

15. A method of preventing splaying of two threadedly engaged components by gripping the engaged threads to each other.

16. An insert element comprising:
a body portion having an outer surface and a first thread disposed on said outer surface, said thread including gripping means for gripping a second thread into which said first thread is threadedly entrained to prevent splaying of said two engaged threads.

17. An external nut having a curvate screw thread.
